# EUROPÄISCHE PATENTSCHRIFT

(11) **EP 1 566 212 B1**
(45) Veröffentlichungstag und Bekanntmachung des Hinweises auf die Patenterteilung: **16.03.2016**
(21) Anmeldenummer: 05009314.5
(22) Anmeldetag: 29.12.2000
(51) Int. Cl.: B01J 19/00, C07C 63/16, C07C 51/265

(54) **Vorrichtung zur Kühlung eines heissen Reaktionsgases, z. B. bei der Herstellung von Phthalsäureanhydrid**
Apparatus for the cooling of hot reaction gases, e.g. for the preparation of phthalic anhydride
Dispositif pour le refroidissement des gazes de réaction chaudes, par example pour la préparation de l'anhydride phtalique

(30) Priorität: 30.12.1999 DE 19963869
(43) Veröffentlichungstag der Anmeldung: 24.08.2005
(62) Teilanmeldung aus: 00991277.5
(73) Patentinhaber: BASF SE, 67056 Ludwigshafen (DE)
(72) Erfinder: Wilke, Alfred, 67166 Otterstadt (DE)

(56) Entgegenhaltungen:
- GB-A- 1 422 516
- US-A- 3 466 300

## Beschreibung

Die Erfindung betrifft eine Vorrichtung zur Kühlung eines heißen Reaktionsgases auf eine vorherbestimmte Eingangstemperatur in einen Abscheider, insbesondere zum Einsatz bei der Herstellung von Phthalsäureanhydrid.

Ausgangspunkt der vorliegenden Erfindung bildet beispielsweise die Herstellung von Phthalsäureanhydrid (PSA) durch Oxidation von o-Xylol. Dabei werden heißes o-Xylol und heiße Luft in Gegenwart eines Katalysators umgesetzt. Das so gebildete PSA verläßt den Reaktor im Gemisch mit Luft als heißes Reaktionsgas und weist eine Temperatur von etwa 360°C auf. Bei PSA-Anlagen mit Nachreaktoren liegt diese Temperatur etwas niedriger. In einem nachgeordneten Abscheider erfolgt dann die Trennung in Roh-PSA und Luft. Vor Eintritt in den oder die Abscheider ist es erforderlich, das heiße Reaktionsgas auf eine vorherbestimmte gewünschte Temperatur, in der Regel etwa 160 bis 175°C, abzukühlen.

Üblicherweise wird hierfür ein komplizierter, mindestens zweistufiger Gaskühler mit mindestens zwei Dampftrommeln und entsprechenden Kühlmittelversorgungen eingesetzt. Dadurch wird das der eigentlichen Reaktion nachgeschaltete Aufbereitungsverfahren des Reaktionsgases aufwendig und kostenintensiv.

Es ist auch schon die Kombination von einem Haupt- mit einem nachgeschalteten Nachreaktor mit oder ohne darin enthaltener Kühlung beschrieben worden; beispielhaft sind die DE-A 197 42 821 oder die DE-A 198 07 018.

In der US 3,466,300 wird ein mehrstufiges (z.B. 14 Stufen) Verfahren zur Herstellung von PSA aus o-Xylol beschrieben, wobei das in die erste Stufe eingeführte Gemisch aus o-Xylol und Luft dadurch vorerhitzt wird, dass es zum Kühlen des die vier ersten Kontaktstufen verlassenden Reaktionsgases eingesetzt wird. Dabei werden Temperatursteigerungen in den ersten Stufen von 50 bis 60°C angestrebt, in den weiteren Stufen von 5 bis 15°C.

Aus der GB 1,422,516 ist ebenfalls ein Verfahren zur Herstellung von PSA aus o-Xylol und Luft bekannt, bei dem bestimmte Bedingungen (Salzbadtemperatur) einzuhalten sind. Es wird zwar auch von der Ausnutzung von entstehender Wärmeenergie (z.B. aus Wärmeaustauschern, Nachreaktoren o.ä.) gesprochen, aber nicht ausschließlich gezielt zur Erhitzung der oder eines der Vorprodukte.

Der Erfindung liegt daher die Aufgabe zugrunde, eine optimierte Vorrichtung zur Kühlung eines heißen Reaktionsgases bereitzustellen, die einen einfacheren und kostengünstigeren Aufbau einer Reaktionsanlage mit geringeren Wartungskosten ermöglicht, und zu einer qualitativ guten und von der Ausbeute her möglichst quantitativen Umsetzung der Edukte, z.B. in einem Verfahren zur Herstellung von Phthalsäureanhydrid, führt.

Gelöst wird die gestellte Aufgabe durch eine Vorrichtung zur Kühlung eines heißen Reaktionsgases auf eine vorherbestimmte Eingangstemperatur zu einem Abscheider nach einer vorhergehenden Reaktion in einem Hauptreaktor und gegebenenfalls einem nachgeordneten Nachreaktor; enthaltend
(a) eine Einrichtung zur Durchführung der Hauptreaktion,
(b) eine Einrichtung zum Wärmetausch,
(c) gegebenenfalls einen der Einrichtung (b) vorgelagerten Nachreaktor mit gegebenenfalls einer Einrichtung zum Zwischenkühlen,
(d) eine Einrichtung zum Abscheiden eines Reaktionsprodukts
und Verbindungsleitungen zu und zwischen den Einrichtungen.
Ferner ist vorgesehen, dass die Einrichtung (b) und/oder der Zwischenkühler ein Gas/Gas-Plattenwärmetauscher ist.

Der Wärmetauscher ersetzt den Gaskühler, wodurch die gesamte Reaktionsanlage einen vereinfachten Aufbau erhält und mit geringerem Kostenaufwand zu warten ist.

Wenn die Verwendung der erfindungsgemäßen Vorrichtung zur Herstellung von PSA dient, wird als ein Reaktand heißes o-Xylol in Gegenwart eines Katalysators und heißer Luft als weiterem Reaktanden umgesetzt. Das durch die Umsetzung entstehende heiße Reaktionsgas besteht im wesentlichen aus PSA und Luft. Da Luft gleichzeitig das Kühlmedium für das heiße Reaktionsgas ist und - wie weiter oben geschildert - mit einer erheblich höheren Temperatur aus dem Wärmetauscher austritt als dies im Stand der Technik möglich ist, kann es direkt in den Reaktor geführt werden. Ein weiterer Vorteil ist, daß in die, die heiße Luft enthaltende Leitung das zunächst eine Temperatur von etwa 30°C aufweisende o-Xylol geführt und gleichzeitig ohne weiteren apparativen und energetischen Aufwand miterwärmt wird. Dennoch resultiert eine gegenüber dem Stand der Technik um etwa 100°C höhere Eintrittstemperatur in den einzigen oder den Haupt-Reaktor, was zu einer besseren Auslastung des Katalysators und einer geringeren Aufheizzone im Reaktor führt.

Demgegenüber war es bisher im Stand der Technik erforderlich, die Luft über einen Vorerwärmer umständlich und energieintensiv vorzuheizen. In diese vorerwärmte Luft wurde das in einem separaten, mit Dampf beheizten Wärmetauscher vorgeheizte flüssige o-Xylol eingespeist. Das Gemisch aus Luft und o-Xylol erreichte aber nicht die nach dem erfindungsgemäßen Verfahren erzielte Eintrittstemperatur in den Reaktor. Außerdem hat sich gezeigt, daß im Reaktor bei der erfindungsgemäßen Verfahrensweise mehr Hochdruckdampf erzeugt werden kann.

Wenngleich die Vorteile des erfindungsgemäßen Verfahrens anhand der Herstellung von PSA dargestellt und seine Vorteile gegenüber dem im Stand der Technik zur Herstellung von PSA bekannten Verfahren geschildert werden, ist der Einsatz der darin erforderlichen Vorrichtung zur Kühlung eines heißen Reaktionsgases nicht auf diese chemische Umsetzung beschränkt. Sie kann ganz allgemein zur Kühlung heißer Reaktionsgase verwendet und für beliebige chemische Umsetzungen herangezogen werden, die in einem Reaktor bei erhöhter Temperatur durchgeführt werden und in denen das Kühlmedium des aus dem Reaktor austretenden Reaktionsgases in dem Wärmetauscher gleichzeitig als ein Reaktand oder Lösungsmittel (flüssig oder gasförmig) bei der Durchführung der chemischen Umsetzung benötigt wird.

In bevorzugten Ausführungsformen ist die Einrichtung (c) ein- oder mehrstückig ausgebildet und von der Einrichtung (b) geht ein Bypass aus, der gegebenenfalls temperaturgesteuert ist.

Als Wärmetauscher wird ein Gas/Gas-Plattenwärmetauscher eingesetzt.

Im folgenden soll die Erfindung anhand von in der Zeichnung dargestellten Ausführungsbeispielen und formen näher erläutert werden.

Es zeigen
- Fig. 1: ein Fließschema des im Stand der Technik bekannten Verfahrens zur Herstellung von PSA
- Fig. 2: ein Fließschema gemäß einer ersten Ausführungsform eines Verfahrens der PSA-Herstellung und der dazu geeigneten Vorrichtung
- Fig. 3: ein Fließschema gemäß einer zweiten Ausführungsform eines Verfahrens der PSA-Herstellung und der dazu geeigneten Vorrichtung
- Fig. 4: ein Fließschema gemäß einer dritten Ausführungsform eines Verfahrens der PSA-Herstellung inkl. Bypassleitung um den Wärmetauscher mit Temperaturregelung
- Fig. 5 a,b: ein Fließchema gemäß einer vierten Ausführunsgform eines Verfahrens der PSA-Herstellung (zwei Plattentauscher inkl. Bypass mit Nachreaktor)
- Fig. 6 a-c: Ausführungsformen der Wärmetauscher mit Nachreaktor(2-er Kombinationen)
- Fig. 7: eine Ausführungsform ohne ersten Wärmetauscher

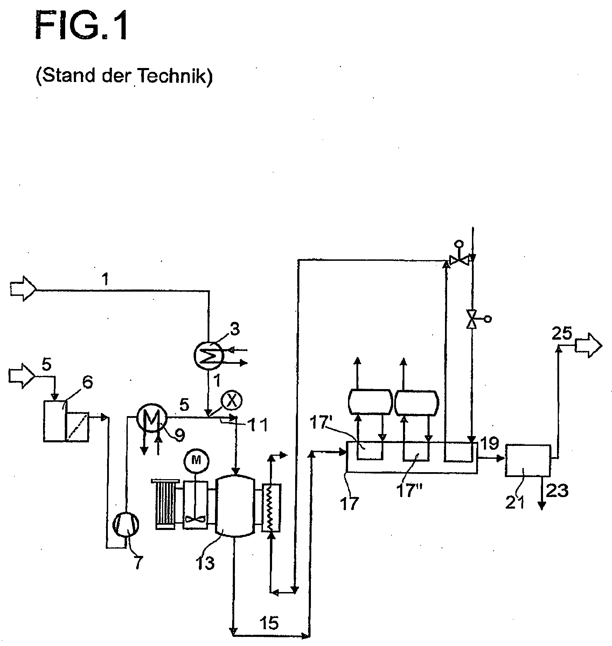

Zunächst wird in Fig. 1 eine Reaktoranlage dargestellt und erläutert, wie sie im Stand der Technik zur Herstellung von PSA bekannt ist. PSA kann bekanntlich durch Oxidation von o-Xylol in Gegenwart eines Katalysators hergestellt werden. Das eingesetzte o-Xylol weist zu Beginn eine Temperatur von etwa 30°C auf und wird über Leitung 1 einem Vorwärmer 3 zugeführt den es mit einer Temperatur von in etwa 135 bis 180°C verläßt. Anschließend wird es in Leitung 5 eingespeist. Über diese Leitung 5 wird Luft zugeführt, die zu Beginn ebenfalls eine Temperatur von etwa 30°C aufweist. Die Luft in Leitung 5 passiert zunächst ein Gebläse 7, wird dabei auf etwa 80 bis 90°C erwärmt und gelangt dann in einen Vorwärmer 9, den sie auf etwa 150 bis 200°C erwärmt wieder verläßt. Zu diesem Zeitpunkt wird das eine Temperatur von etwa 135 bis 180°C aufweisende o-Xylol aus Leitung 3 in Leitung 5 eingespeist. Das Luft/o-Xylol-Gemisch in Leitung 5 passiert eine Mantelleitungsheizung 11, die dazu dient, die Temperatur des Luft/o-Xylol-Gemisches bei etwa 160 bis 175°C zu halten und erreicht so den Reaktor 13 für die chemische Umsetzung.

Der Reaktor 13 stellt einen Rohrreaktor dar, in dessen Röhren sich der für diese Oxidationsreaktion erforderliche Katalysator, z.B. Cs dotiertes Vanadium/Titan-Oxid auf einem Träger wie Steatit, befindet.

Nach erfolgter chemischer Umsetzung verläßt das Reaktionsgas mit einer Temperatur von etwa 350 bis 375°C den Reaktor 13 über Leitung 15, es besteht im wesentlichen aus PSA und Luft; Nebenbestandteile sind z.B. Phthalid und Maleinsäureanhydrid. Von dort gelangt es zu einem zweistufigen Gaskühler 17, der zwei Dampftrommeln 17', 17" aufweist und wird auf eine Temperatur von etwa 160 bis 175°C abgekühlt. Damit weist das Reaktionsgas die vorherbestimmte günstige Temperatur auf, um über Leitung 19 zu einem Abscheider 21 geführt zu werden. Aus dem Abscheider 21 wird über Leitung 23 Roh-PSA entnommen und über Leitung 25 das Abgas einer Verbrennung oder Wäsche zugeführt.

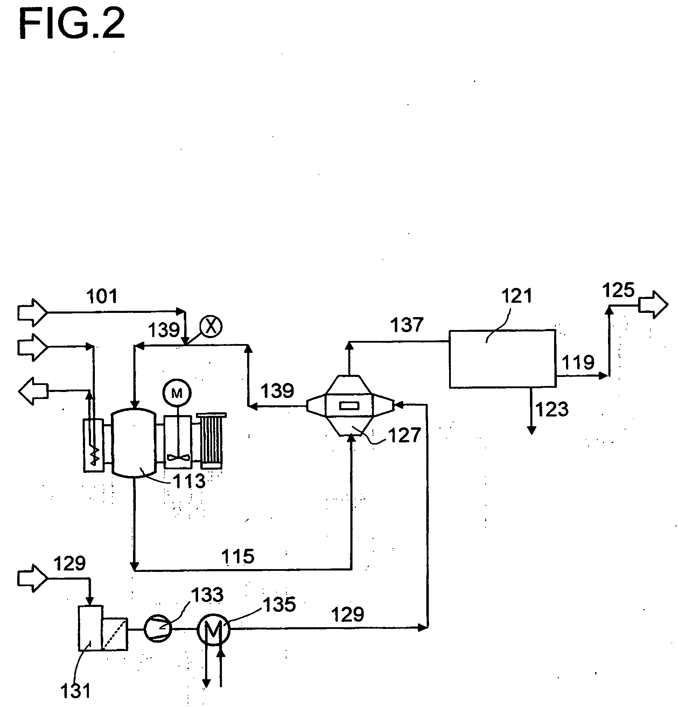

Demgegenüber ist in Fig. 2 ein Verfahren zur PSA-Herstellung mit dazu geeigneter erfindungsgemäßer Vorrichtung dargestellt. Dabei werden solche Vorrichtungsmerkmale, die den in Fig. 1 bereits dargestellten entsprechen, mit den selben, jedoch um 100 erweiterten Bezugszahlen versehen.

Für die Durchführung der chemischen Umsetzung wird anstelle des zweistufigen Gaskühlers ein einstufiger Wärmetauscher, hier ein Gas/Gas-Plattenwärmetauscher 127 verwendet. Dadurch kann die Reaktionsführung gleich mehrfach verändert werden und es ist daher vorteilhaft, die erfindungsgemäße Durchführung der Umsetzung ausgehend von dem Gas/Gas-Plattenwärmetauscher 127 zu beschreiben.

Dem Gas/Gas-Plattenwärmetauscher 127 wird aus dem Reaktor 113 strömendes Reaktionsgas, mit einer Temperatur von etwa 350 bis 375°C über Leitung 115 zugeführt. Gleichzeitig fließt über Leitung 129 ein Kühlmittel in Form von Luft mit einer Temperatur von etwa 90°C in den Plattenwärmetauscher 127 ein. Diese Kühlmittel-Luft weist zunächst eine Temperatur von etwa 30°C auf und wird über einen Luftfilter 131 geleitet, um anschließend mittels eines Gebläses 133 und eines Anfahrerhitzers 135 auf eine Temperatur von 90°C erwärmt zu werden. In dem Plattenwärmetauscher 127 findet nunmehr ein Wärmeaustausch zwischen dem heißen Reaktionsgas und der Kühlmittel-Luft statt. Das Reaktionsgas verläßt den Plattenwärmetauscher 127 anschließend über Leitung 137 mit einer Temperatur von etwa 160 bis 175°C. Damit weist das Reaktionsgas die vorherbestimmte günstige Temperatur auf, um dem Abscheider 121 zugeführt und anschließend auf die schon beschriebene Weise in Roh-PSA und Abgas getrennt zu werden. Durch den Wärmeaustausch zwischen dem heißen Reaktionsgas und der Kühlmittel-Luft wird letztere auf etwa 330°C erwärmt und kann jetzt über Leitung 139 dem Reaktor 113, der gleichartig zum bereits beschriebenen Stand der Technik ausgebildet ist, als einer der Reaktanden für die chemische Umsetzung zugeführt werden.

D. h. gegenüber dem bisher bekannten Verfahren wird die Kühlmittel-Luft um etwa 150° höher aufgeheizt, wodurch ihr erneuter Einsatz als ein Reaktand der chemischen Umsetzung ohne die bisher notwendige energieintensive Vorwärmung dieses Reaktanden über einen Vorwärmer (z.B. 9) möglich und sinnvoll ist.

Gleichzeitig entfällt aber auch die bisher notwendige Vorwärmung des o-Xylols über einen weiteren Vorwärmer (z.B. 3).

o-Xylol, das über Leitung 101 zugeführt wird, gelangt direkt in die die heiße Kühlmittel-Luft aufweisende Leitung 139 und es bildet sich das für die Umsetzung zu PSA benötigte o-Xylol/Luft-Gemisch. Durch die Zuführung des etwa eine Temperatur von 30°C aufweisenden o-Xylol kühlt sich das o-Xylol/Kühlmittel-Luft Gemisch zwar auf etwa 280°C ab. Diese Temperatur, mit der es dann in den Reakter 113 eintritt, liegt aber immer noch gut 100°C höher, als dies nach der bekannten Verfahrensweise möglich ist. Dadurch kann der Katalysator im Reaktor 113 besser ausgenutzt und mehr Hochdruckdampf erzeugt werden. Da die separate o-Xylol-Vorwärmung über einen Vorwärmer und z.B. die Mantelheizung 11 der in Fig. 1 zum Reaktor führenden Leitung 5 entfallen, wird der Anlagenaufbau einfacher und kostengünstiger, was ebenso für die Wartung der Anlage gilt.

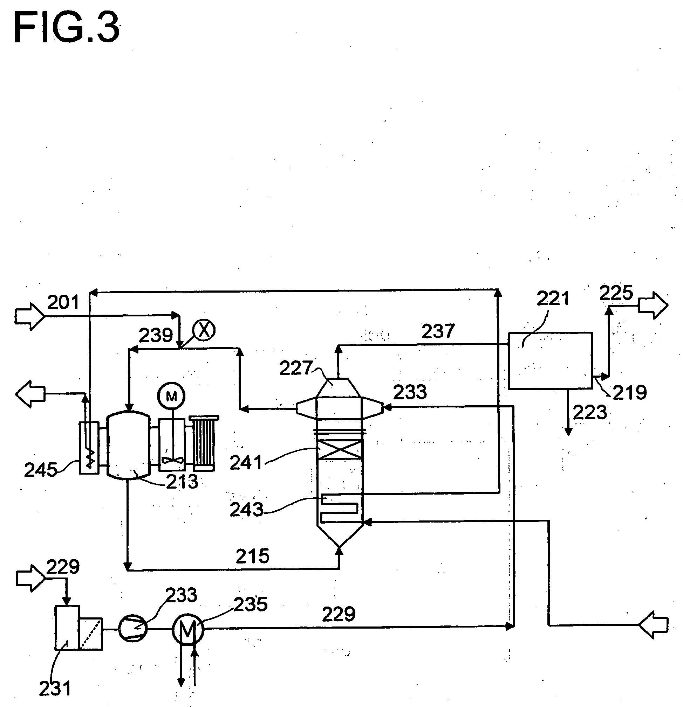

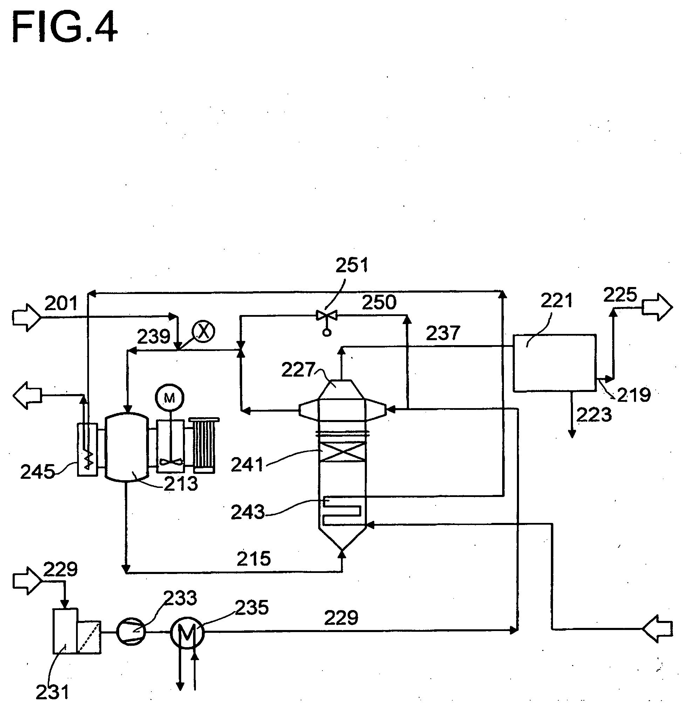

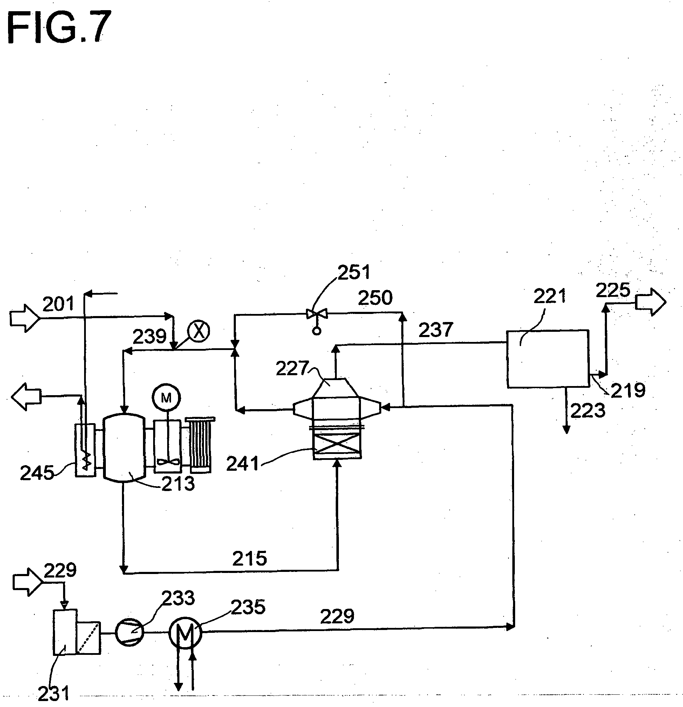

In Fig. 3 ist eine weitere Ausführungsform eines Verfahrens zur PSA-Herstellung und einer dazu geeigneten erfindungsgemäßen Vorrichtung dargestellt. Dabei werden die zu der Ausführungsform gemäß Fig. 2 gleichen Vorrichtungsmerkmale mit entsprechend gleichen, aber um hundert erweiterten Bezugszahlen versehen; diese Art der Bezugszahlen wird dann in den weiteren Figuren (d.h. Zahlen ab 200) beibehalten. Fig. 4 zeigt eine zu Fig. 3 analoge Anordnung mit einer Bypassleitung 250 einschl. einer Temperaturregelung 251, hingegen Fig. 7 eine sich von Fig. 4 unterscheidende Anordnung ohne den Zwischenkühler 243.

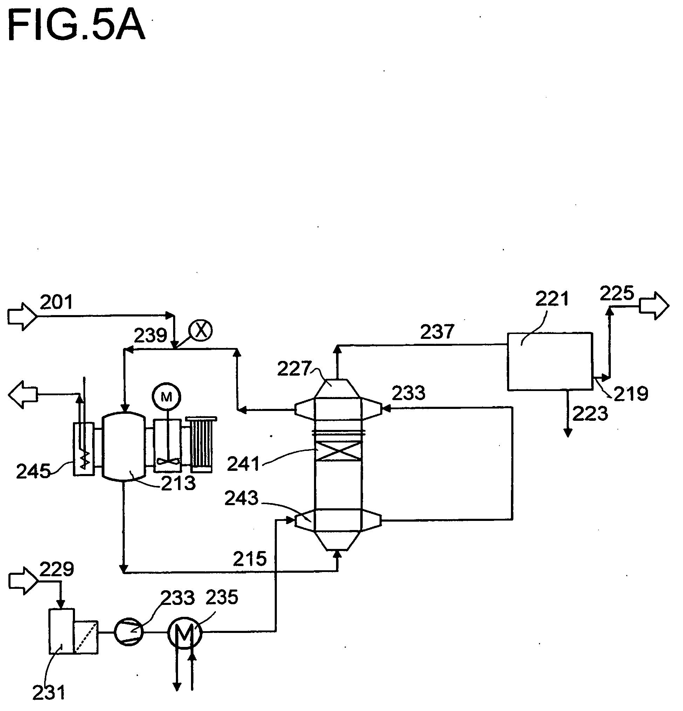

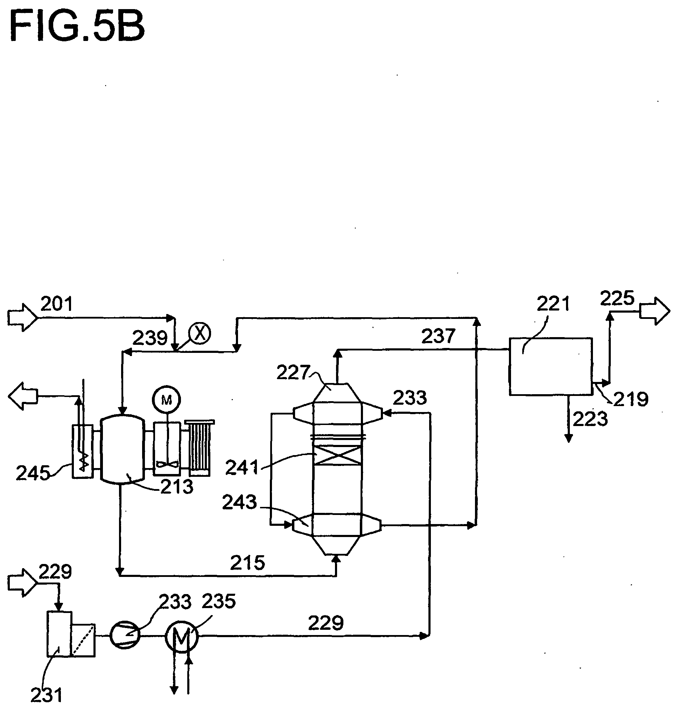

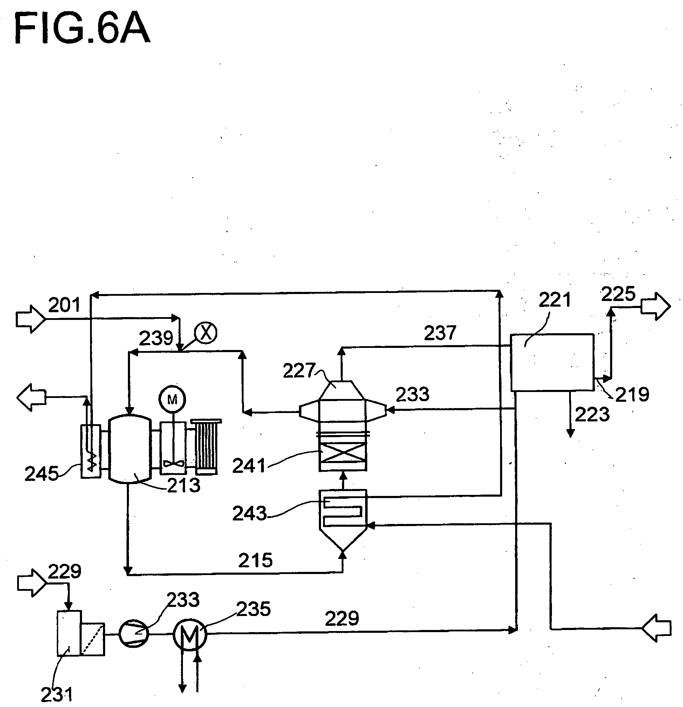

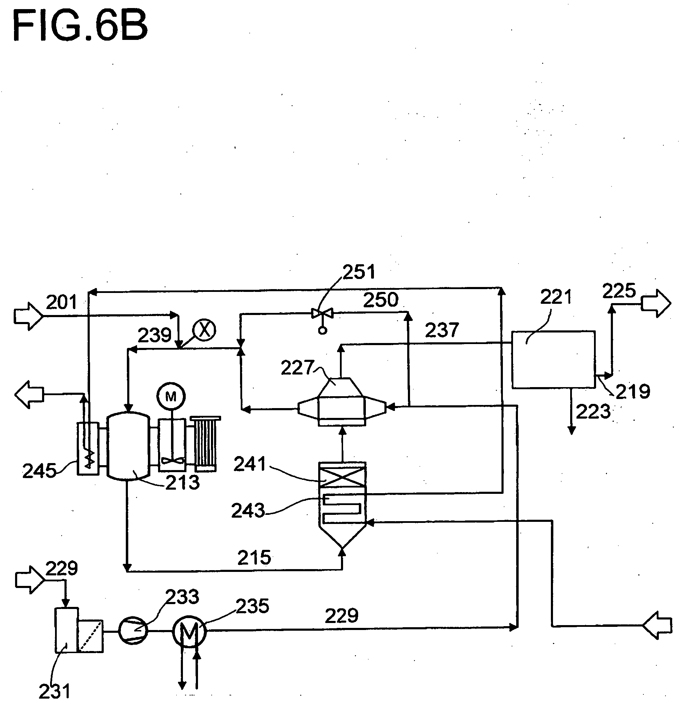

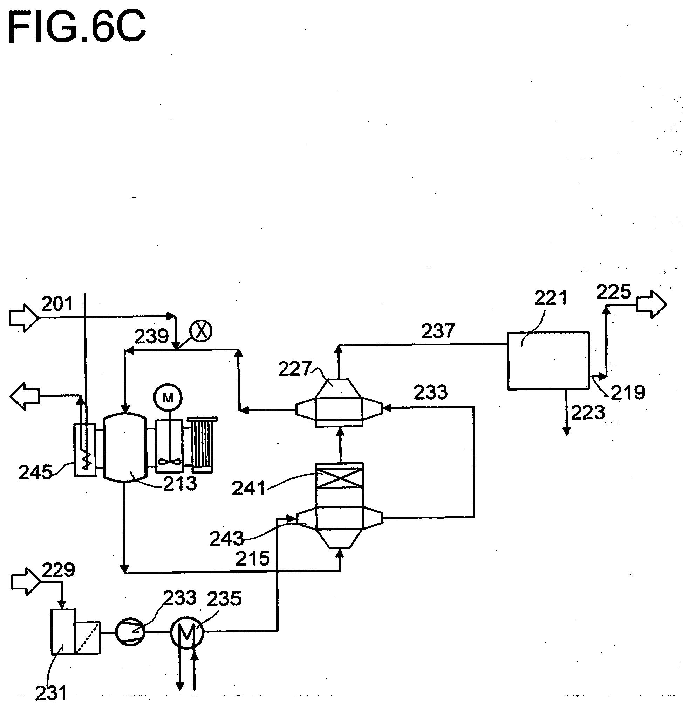

Die Ausführungsform gemäß Fig. 3 unterscheidet sich von der Ausführungsform gemäß Fig. 2 grundsätzlich nur dadurch, daß dem Reaktor 213 ein Nachreaktor 241 mit einem gleichen oder von dem im Hauptreaktor 213 verschiedenen Katalysator nachgeschaltet ist; ein geeigneter Katalysator für den Nachreaktor ist beispielsweise ein undotiertes Vanadium/Titan-Oxid auf einem Träger wie Siliciumcarbid. Da der Nachreaktor 241 eine niedrigere Eingangstemperatur aufweisen soll als das Reaktionsgas nach seinem Austritt aus dem eigentlichen Reaktor 213 aufweist, ist dem Nachreaktor 241 ein Zwischenkühler 243 vorgeschaltet. Der Zwischenkühler 243 kann dabei als Brauchwasservorerwärmer für einen Salzbadkühler 245, der dem Reaktor 213 zugeordnet ist, ausgeführt werden. Er kann aber genauso als Vorerwärmer für die Kühlmittel-Luft, z. B. vom Typ eines weiteren Gas/Gas-Plattenwärmetauschers oder eines anderen Wärmeaustauschertyps, ausgeführt werden. Dies ist in Fig. 5 a und b dargestellt, wobei dann z.B. die den einen Plattenwärmetauscher 243 (Zwischenkühler) verlassende Kühlmittel-Luft dem zweiten Plattenwärmetauscher 227 als Kühlmittel-Luft (Fig. 5a) - oder auch in umgekehrter Folge (Fig. 5b) - zugeleitet werden kann. Dabei sind der Zwischenkühler 243, der Nachreaktor 241 und der Gas/Gas-Plattenwärmetauscher 227 in der Ausführungsform gemäß Fig. 3 in einem gemeinsamen Gehäuse angeordnet. Es hat sich jedoch gezeigt, daß es zur Optimierung der Reaktionserfordernisse auch sinnvoll sein kann, den Zwischenkühler 243, den Nachreaktor 241 und/oder den Gas/Gas-Plattenwärmetauscher 227 in voneinander getrennten Gehäusen anzuordnen. Dabei können alle drei Vorrichtungen in jeweils eigenen Gehäusen oder jeweils zwei der genannten Vorrichtungen wiederum zusammen in einem Gehäuse angeordnet sein. Diese Varianten sind in Fig. 6a (Trennung des Zwischenkühlers 243 von Nachreaktor 241 und Wärmetauscher 227), 6b (Trennung des Wärmetauschers 227 von Zwischenkühler 243 und Nachreaktor 241) und 6c (wie 6b ohne Bypass 250 aber 243 als Gas/Gas-Plattenwärmetauscher), jeweils mit einer Verbindung zwischen den Bauteilen dargestellt.

## Patentansprüche

1. Vorrichtung zur Kühlung eines heißen Reaktionsgases auf eine vorherbestimmte Eingangstemperatur zu einem Abscheider nach einer vorhergehenden Reaktion in einem Hauptreaktor und gegebenenfalls einem nachgeordneten Nachreaktor; enthaltend
(a) eine Einrichtung zur Durchführung der Hauptreaktion (113, 213),
(b) eine Einrichtung zum Wärmetausch (127, 227),
(c) gegebenenfalls einen der Einrichtung (b) vorgelagerten Nachreaktor (241) mit gegebenenfalls einer Einrichtung zum Zwischenkühlen (243),
(d) einer Einrichtung zum Abscheiden eines Reaktionsprodukts (121, 221) und Verbindungsleitungen zu und zwischen den Einrichtungen (a) bis (d),
**dadurch gekennzeichnet, dass** die Einrichtung (b) und/oder der Zwischenkühler (241) ein Gas/Gas-Plattenwärmekühler ist.

2. Vorrichtung nach Anspruch 1, **dadurch gekennzeichnet, dass** die Einrichtung (c) ein- oder mehrstückig ausgebildet ist.

3. Vorrichtung nach Anspruch 1 oder 2, **dadurch gekennzeichnet, dass** von der Einrichtung (b) ein über eine Leitung (139, 239) zum Reaktor (113, 213) führender Bypass (250) ausgeht, der gegebenenfalls temperaturgesteuert (251) ist.

4. Verwendung der Vorrichtung nach einem der Ansprüche 1 bis 3 in einem Verfahren zur Herstellung von Phthalsäureanhydrid.

## Claims

1. An apparatus for cooling a hot reaction gas to a previously determined inlet temperature to a separator after a preceding reaction in a main reactor and optionally a downstream secondary reactor; containing
(a) an appliance for carrying out the main reaction (113, 213),
(b) an appliance for heat exchange (127, 227),
(c) optionally a secondary reactor (241) upstream of the appliance (b) having optionally an appliance for intermediate cooling (243),
(d) an appliance for separating off a reaction product (121, 221)
and connection lines to and between the appliances (a) to (d),
wherein the appliance (b) and/or the intercooler (241) is a gas/gas plate heat cooler.

2. The apparatus according to claim 1, wherein the appliance (c) is designed so as to be one piece or multipiece.

3. The apparatus according to claim 1 or 2, wherein a bypass (250) leading via a line (139, 239) to the reactor (113, 213) departs from the appliance (b), which bypass is optionally under temperature control (251).

4. The use of the apparatus according to any one of claims 1 to 3 in a process for preparing phthalic anhydride.

## Revendications

1. Dispositif de refroidissement d'un gaz de réaction chaud à une température d'entrée prédéterminée dans un séparateur suite à une réaction préalable dans un réacteur principal et éventuellement un post-réacteur disposé en aval ; comprenant
(a) un dispositif pour mettre en oeuvre la réaction principale (113, 213),
(b) un dispositif pour l'échange de chaleur (127, 227),
(c) éventuellement un post-réacteur (241) monté en amont du dispositif (b), avec éventuellement un dispositif pour le refroidissement intermédiaire (243),
(d) un dispositif pour séparer un produit de réaction (121, 221)
et des conduites de liaison vers et entre les dispositifs (a) à (d),
**caractérisé en ce que** le dispositif (b) et/ou le refroidisseur intermédiaire (241) est un refroidisseur de chaleur à plaques gaz/gaz.

2. Dispositif selon la revendication 1, **caractérisé en ce que** le dispositif (c) est réalisé d'une seule pièce ou en plusieurs parties.

3. Dispositif selon la revendication 1 ou 2, **caractérisé en ce qu'**une dérivation (250) conduisant par le biais d'une conduite (139, 239) au réacteur (113, 213), qui est éventuellement régulée en température (251), part du dispositif (b) .

4. Utilisation du dispositif selon l'une quelconque des revendications 1 à 3 dans un procédé de fabrication d'un hydrure d'acide phtalique.
